# EUROPEAN PATENT SPECIFICATION

(11) **EP 4 091 551 B1**
(45) Date of publication and mention of the grant of the patent: **08.05.2024**
(21) Application number: 21912549.9
(22) Date of filing: 25.02.2021
(51) Int. Cl.: A61B 17/04, A61F 2/08

(54) **LOOP AND MANUFACTURING METHOD THEREFOR**
SCHLEIFE UND HERSTELLUNGSVERFAHREN DAFÜR
BOUCLE ET SON PROCÉDÉ DE FABRICATION

(30) Priority: 31.12.2020 CN 202011641868
(43) Date of publication of application: 23.11.2022
(73) Proprietor: Shanghai Orthopair Medical Co., Ltd., Shanghai 201201 (CN)
(72) Inventor: YUAN, Zheng, Shanghai 201201 (CN); NIE, Jiali, Shanghai 201201 (CN); WANG, Yuanqiang, Shanghai 201201 (CN)
(74) Representative: Bayramoglu et al.
(86) International application number: PCT/CN2021/077882
(87) International publication number: WO 2022/141779

(56) References cited:
- WO-A1-2020/109817
- CN-A- 108 392 235
- CN-A- 108 472 032
- CN-A- 109 646 065
- CN-A- 110 680 427
- CN-U- 210 673 419
- KR-A- 20160 087 580
- US-A- 5 693 060
- US-A1- 2008 177 304
- US-A1- 2017 202 658
- US-A1- 2020 315 774
- US-B1- 7 875 043

## Description

### TECHNICAL FIELD

The present invention relates to the field of medical devices, and more specifically, to a loop and a manufacturing method therefor.

### BACKGROUND ART

Loop is used for fixing ligaments or tendons and bones in orthopedic reconstruction, and is commonly used for anterior and posterior cruciate ligament reconstruction of the knee joints, acromioclavicular joint dislocation fixation and like. For example, in cruciate ligament surgery, bone tunnels need to be drilled in femur and tibia respectively, and a ligament graft without bone blocks at both ends is secured in the bone tunnels of the femur and tibia using a titanium plate with a loop and screws.

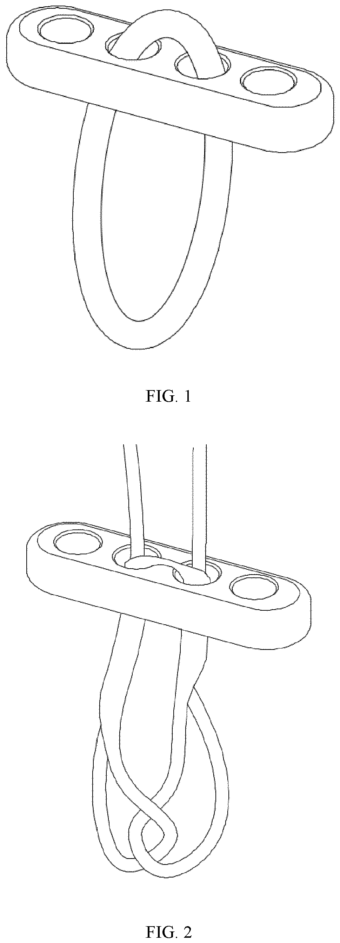

In the cruciate ligament reconstruction of the knee joints, the ligament graft should fill the femoral tunnel as completely as possible, and existing titanium plates with the loop are divided into a length-fixed titanium plate with a loop and an adjustable titanium plate with a loop. FIG. 1 is a structure diagram of a fixed-length titanium plate with a loop in the prior art. Referring to FIG. 1, a coil of the fixed-length titanium plate with the loop is usually of fixed specifications in different sizes, has no ability of adjusting the length of the coil, needs measurement and calculation before use, and is hard to completely fill the femoral tunnel, which may increase unnecessary surgery time and has surgical risks, as well as requires more space in the operating room for storage. FIG. 2 is a structure diagram of an adjustable titanium plate with a loop in the prior art. Referring to FIG. 2, the adjustable titanium plate with the loop can solve the problem of the fixed-length titanium plate with the loop. However, the product currently available on the market is not conducive to surgery due to fact that the reaction force of tendon and other grafts makes the coil easily retreat; and the processing technology is complicated, and requires special equipment for winding and weaving to meet the same mechanical strength as the fixed-length titanium plate with the loop, resulting in high investment cost.

The patent application with publication number of CN108392235A discloses a loop used for a titanium plate with a loop, and in particular a loop for a titanium plate with the loop and a weaving process for the loop. The loop is woven to form a continuous ring by a cored woven rope, the cross section of the cored woven rope is oval, and a core yarn is arranged in the cored woven rope. The cored woven rope comprises a woven segment A, a woven segment B and a non-woven segment, wherein the woven segment A and the woven segment B are respectively two free ends of the cored woven rope. One end of the non-woven segment is connected to the woven segment A, and the other end of the non-woven segment is connected to the woven segment B. An end A of the woven segment A threads in a hollow interior of the woven segment B to form a nesting portion A, and an end B of the woven segment B threads in a hollow interior of the woven segment A to form a nesting portion B. Compared with direct adhesive bonding or knotting fixation, the loop can overcome the defects that an existing medical suture loop is not firm in fixation and the like, and irritation to tissues is smaller. However, under the reaction force of a graft, an outer line sleeve of the cored woven rope is easy to deform, and a line core is relatively small in deformation, which is not conducive to the surgery; and the loop in this invention belongs to fixed-length loops, which has no ability of adjusting the length of the coil and requires measurement and calculation before use; there is only one suture segment for fixing the graft, so that the material requirement of the suture is higher to meet the medical mechanical strength; and meanwhile, the cored woven rope is too thick to be convenient for clinical use.

The patent publication with publication number of US2017202658A1 discloses using flexible implantable bodies in conjunction with a suture filament or repair construct formed to have adjustable coils for use in maintaining a location of a graft with respect to a bone. The surgical implant in question comprises; a flexible filament body and a suture filament extending through the flexible filament body at two or more separate locations on the flexible filament body to form one or more coils such that a portion of each coil is disposed on a top side of the body and a portion of each coil is disposed on a bottom side of the body with each coil defining an opening thereof, the suture filament also having a slidable portion formed therefrom such that movement of the slidable portion towards and away from the flexible filament body causes a size of at least one opening of the one or more coils to change, and a first location and a second location of the two or more separate locations on the flexible filament body being located on opposed sides of the flexible filament body along a length of the body, with the slidable portion of the suture filament being disposed between the first and second locations.

The patent application with publication number of US2020315774A1 discloses a medical implant, comprising at least a first cortical fixation device for fixing a ligament or tendon graft in a desired position, wherein said first cortical fixation device comprises a body having a front side and a rear side, which rear side faces away from the front side, and wherein said body comprises a first and an adjacent second through-hole, which through-holes extend from the front side to the rear side of said body. According to the invention the medical implant further comprises a first suture thread extending through the through-holes and forming a twisted loop (104) and a non-twisted loop on the front side of the body, and a sliding knot on the rear side of the body, and wherein the medical implant comprises a second suture thread extending through the sliding knot.

The patent publication with publication number of US5693060A discloses a suture securing device and method. The suture device in question includes a surgically implantable body having a tissue abutting side and a suture loop side, and a passage through the body having an opening adjacent the tissue abutting side and another opening adjacent the suture loop side. When a loop of suture is positioned through the passage from the tissue abutting side and emerges from the suture loop side and a free portion of suture is passed through the loop, the loop clamps upon the free portion when the loop is retracted back into the passage.

The devices disclosed above-mentioned documents still inadequate in terms of meeting clinical requirements of structural mechanics. Specifically, they may be difficult to be adapted for the grafts with different pulling force.

Therefore, it is necessary to design a novel loop and a manufacturing method thereof to avoid the problems in the prior art.

### SUMMARY

An objective of the present invention is to provide a loop and a manufacturing method thereof to solve the problems that in the prior art, the coil length in a fixed-length titanium plate with a loop cannot be adjusted while the coil of an adjustable titanium plate with a loop is easy to retreat under the reaction force of a graft, leading to inconvenience in the surgery.

To achieve the objective, a loop is provided, comprising a fixing plate and at least one suture, wherein the fixing plate is provided with at least two threading holes, the suture is wound to form a first coil and a second coil, and the first coil and the second coil are provided on a first side of the fixing plate; two ends of the first coil are respectively a first end and a second end, and two ends of the second coil are respectively a third end and a fourth end; the first end and the third end pass through different threading holes and are connected on a second side of the fixing plate to form a first fixing portion, the second end forms at least one second fixing portion on the second side of the fixing plate by passing through the plurality of threading holes, the second end, after forming the second fixing portion, is wound around part of the first coil and part of the second coil on the first side of the fixing plate to form an adjusting portion.

The loop disclosed by the present invention has the following beneficial effects: the suture is wound to form the first coil and the second coil, and the first coil and the second coil are provided on the first side of the fixing plate, so that the first coil and the second coil can share the pulling force of the graft when fixing the graft, the overall stress strength of the loop is increased, and the loop is firmer, can meet clinical requirements of structural mechanics, and can be suitable for the grafts with different pulling force; the two ends of the first coil are respectively the first end and the second end, and the two ends of the second coil are respectively the third end and the fourth end; the first end and the third end pass through different threading holes and are connected on the second side of the fixing plate to form the first fixing portion, so that the first fixing portion can automatically slide on the fixing plate under the action of the graft to adjust the length of the first coil and the length of the second coil so as to make the length of the first coil the same as the length of the second coil, thus guaranteeing that the first coil and the second coil can share the pulling force of the graft, no redundant calculation is required during surgery, the surgery time is shortened, and the surgical risk is reduced; the second end forms at least one second fixing portion on the second side of the fixing plate by passing through the plurality of threading holes, and the second end, after forming the second fixing portion, is wound around part of the first coil and part of the second coil on the first side of the fixing plate to form the adjusting portion, so that the first coil and the second coil are connected to the fixing plate, and the length of the first coil and the length of the second coil can be adjusted by adjusting the adjusting portion to enable the fourth end to slide and stretch under the action of artificial external force, i.e., the total length of the first coil and the second coil, no redundant calculation is required during surgery, and the surgery time is shortened; when the graft is fixed by the first coil and the second coil, under the action of the pulling force of the graft, the adjusting portion is pulled by the second end to contract and lock the first coil and the second coil, so that the fourth end cannot slide and stretch to adjust the length of the first coil and the length of the second coil, and the second end can automatically lock the first coil and the second coil without external force operation, the first coil and the second coil can be effectively prevented from retreating, the stability and safety of the loop in clinical use are guaranteed, the smooth surgery is ensured, and the surgical risk is reduced.

Preferably, the adjusting portion comprises at least one of a wound coil and a movable suture knot. The beneficial effects include: the length of the first coil and the length of the second coil can be conveniently adjusted by adjusting the adjusting portion to enable the fourth end to slide and stretch under the action of artificial external force, and when the graft is fixed by the first coil and the second coil, the second end can automatically lock the first coil and the second coil without external force operation under the action of the pulling force of the graft, the first coil and the second coil can be effectively prevented from retreating, the stability and safety of the loop in clinical use are guaranteed, the smooth surgery is guaranteed, and the surgical risk is reduced.

Preferably, the adjusting portion comprises at least one wound coil, the second end, after forming the second fixing portion, is wound around part of the first coil and part of the second coil on the first side of the fixing plate so as to form the wound coil. The beneficial effects include: the wound coil is more uniformly stressed, so that the wound coil can be pulled easier to contract and lock the first coil and the second coil when the second end is subjected to the action of the pulling force of the graft.

Preferably, the adjusting portion further comprises a first movable suture knot, the second end, after forming the wound coil, is further wound around part of the first coil and part of the second coil so as to form the first movable suture knot. The beneficial effects include: the wound coil is prevented from loosening, and the first coil and the second coil are further locked to prevent the first coil and the second coil from retreating.

Preferably, the adjusting portion comprises at least one second movable suture knot, the second end, after forming the second fixing portion, is wound around part of the first coil and part of the second coil on the first side of the fixing plate so as to form the second movable suture knot. The beneficial effects include: when subjected to the action of the pulling force of the graft, the second end pulls the second movable suture knot to contract to make part of the first coil and part of the second coil deform, thus increasing the friction force between the second movable suture knot and part of the first coil as well as part of the second coil, and effectively preventing the first coil and the second coil from retreating under the action of the pulling force of the graft; and the second movable suture knot has a better anti-retreating effect for the first coil and the second coil.

Preferably, the second end, after forming the adjusting portion, is compressed between any one of the first fixing portion and the second fixing portion and the fixing plate. The beneficial effects include: when the graft is fixed by the first coil and the second coil, the second fixing portion or the first fixing portion can automatically contract to compress and fix the second end, thus effectively preventing the adjusting portion from loosening, and making the first coil and the second coil have better anti-retreating effect.

Preferably, the adjusting portion is arranged close to the fourth end, and the adjusting portion is arranged to be in contact with an end face of the first side of the fixing plate. The beneficial effects include: on the one hand, the fourth end can be better fixed to prevent the fourth end from falling to the first side of the fixing plate; on the other hand, when the length of the first coil and the length of the second coil are adjusted by adjusting the adjusting portion to make the fourth end slide and stretch under the artificial external force, the adjusting portion is in contact with the end face of the first side of the fixing plate, i.e., the adjusting portion directly abuts against the end face of the first side of the fixing plate, the operation of pulling the adjusting portion to move to the end face of the first side of the fixing plate is avoided, so that the medical staff can rapidly and accurately judge the length of the first coil and the length of the second coil, and the medical staff is prevented from misjudging.

Preferably, the first coil and the second coil are woven by one continuous suture of at least one suture passing through the threading holes of the fixing plate. The beneficial effects include: a suture segment between the first coil and the second coil is continuous, firmer and more durable, thus preventing the joint of the first coil and the second coil, i.e., the first fixing portion, from being broken to affect the use.

Preferably, the first coil and the second coil are respectively woven by two sutures of at least one suture passing through the threading holes of the fixing plate. The beneficial effects include: the weaving of the loop is simple and convenient.

Preferably, the fourth end is compressed between any one of the first fixing portion and the second fixing portion and the fixing plate. The beneficial effects include: when the graft is fixed by the first coil and the second coil, the first fixing portion or the second fixing portion can automatically contract to compress and fix the fourth end, thus effectively preventing the fourth end from falling to the first side of the fixing plate and preventing the fourth end from sliding under the acting force of the graft; the first coil and the second coil can be further prevented from retreating, and the anti-retreating effect is better.

Preferably, a manufacturing method of a loop is further provided, comprising:
S0, providing a fixing plate and at least one suture, wherein the fixing plate is provided with at least two threading holes; and
S1, winding the suture to form a first coil and a second coil, and providing the first coil and the second coil on a first side of the fixing plate, and threading a first end of the first coil and a third end of the second coil through different threading holes and connecting the first end of the first coil to the third end of the second coil on a second side of the fixing plate to form a first fixing portion, and after threading the second end of the first coil through the plurality of threading holes to form at least one second fixing portion on the second side of the fixing plate, winding the second end around part of the first coil and part of the second coil at the first side of the fixing plate to form an adjusting portion, thus forming the loop.

The manufacturing method for the loop has the following beneficial effects: the suture is wound to form the first coil and the second coil, the first coil and the second coil are provided on the first side of the fixing plate, so that the first coil and the second coil can share the pulling force of the graft when fixing the graft, the overall stress strength of the loop is increased, and the loop is firmer, can meet clinical requirements of structural mechanics, and can be suitable for the grafts with different pulling force; the two ends of the first coil are respectively the first end and the second end, and the two ends of the second coil are respectively the third end and the fourth end; the first end and the third end pass through different threading holes and are connected on the second side of the fixing plate to form the first fixing portion, so that the first fixing portion can automatically slide on the fixing plate under the acting force of the graft to adjust the length of the first coil and the length of the second coil, and the length of the first coil is the same as the length of the second coil, thus guaranteeing that the first coil and the second coil can share the pulling force of the graft, and no redundant calculation is required during surgery, the surgery time is shortened, and the surgical risk is reduced; the second end forms at least one second fixing portion on the second side of the fixing plate by passing through the plurality of threading holes, and the second end, after forming the second fixing portion, is wound around part of the first coil and part of the second coil on the first side of the fixing plate to form the adjusting portion, so that the first coil and the second coil are connected to the fixing plate, and the length of the first coil and the length of the second coil can be adjusted by adjusting the adjusting portion to enable the fourth end to slide and stretch under the action of artificial external force, i.e., the total length of the first coil and the second coil, no redundant calculation is required during surgery, and the surgery time is shortened; when the graft is fixed by the first coil and the second coil, under the action of the pulling force of the graft, the second end pulls the adjusting portion to contract and lock the first coil and the second coil, so that the fourth end cannot slide and stretch to adjust the length of the first coil and the length of the second coil, and the second end can automatically lock the first coil and the second coil without external force operation, the first coil and the second coil are effectively prevented from retreating, the stability and safety of the loop in clinical use are guaranteed, the smooth surgery is ensured, and the surgical risk is reduced.

Preferably, when the first coil and the second coil are woven by one continuous suture of at least one suture passing through the threading holes of the fixing plate, the step S1 specifically comprises:
S101, winding the continuous suture to form the first coil and the second coil, and providing the first coil and the second coil on the first side of the fixing plate, and forming a first fixing portion on the second side of the fixing plate; and
S102, after threading the second end of the first coil through the plurality of threading holes to form at least one second fixing portion on the second side of the fixing plate, winding the second end around part of the first coil and part of the second coil at the first side of the fixing plate to form an adjusting portion. The beneficial effects include: a suture segment between the first coil and the second coil is continuous and is firmer and more durable, thus preventing the joint of the first coil and the second coil from being broken to affect the use.

Preferably, when the first coil and the second coil are respectively woven by two sutures of at least one suture passing through the threading holes of the fixing plate, the step S1 specifically comprises:
S111: winding one of the sutures to form the first coil and winding the other of the sutures to form the second coil, and providing the first coil and the second coil on the first side of the fixing plate;
S112: after threading the first end of the first coil and the third end of the second coil through different threading holes, connecting the first end of the first coil to the third end of the second coil on the second side of the fixing plate to form the first fixing portion; and
S113, after threading the second end of the first coil through the plurality of threading holes to form at least one second fixing portion on the second side of the fixing plate, winding the second end around part of the first coil and part of the second coil on the first side of the fixing plate to form the adjusting portion. The beneficial effects include: the weaving of the loop is simple and convenient.

### BRIEF DESCRIPTION OF THE DRAWINGS

FIG. 1 is a structure diagram of a fixed-length titanium plate with a loop in the prior art;
FIG. 2 is a structure diagram of an adjustable titanium plate with a loop in the prior art;
FIG. 3 is a structure diagram of a first loop of an embodiment of the present invention;
FIG. 4 is a sectional diagram of A in FIG. 3 along a direction of the first and second coils being wound;
FIG. 5 is a diagram of a use state of a loop in an embodiment of the present invention;
FIG. 6 is a structure diagram of a second loop in an embodiment of the present invention;
FIG. 7 is a structure diagram of a third loop in an embodiment of the present invention;
FIG. 8 is a structure diagram of a fixing plate in an embodiment of the present invention;
FIG. 9 is a structure diagram formed in a manufacturing process of a loop in an embodiment of the present invention;
FIG. 10 is another structure diagram formed in a manufacturing process of a loop in an embodiment of the present invention.

### DETAILED DESCRIPTION

In order to make objectives, technical solutions, and advantages of the present invention clearer, the technical solutions in the present invention are described clearly and completely in the following with reference to accompanying drawings in the embodiments of the present invention. Apparently, the described embodiments are only part rather than all of the embodiments of the present invention. Based on the embodiments of the present invention, all the other embodiments obtained by those of ordinary skill in the art without inventive effort are within the scope of the present invention. Unless otherwise mentioned, all technical and scientific terms used herein have the same meaning as commonly understood by one of ordinary skill in the art to which the present invention belong. As used herein, "comprising" and like words mean that elements or objects appearing before the word encompass elements or objects listed after the word and equivalents thereof, without excluding other element or objects.

To solve the problem in the prior art, a loop is provided in an embodiment of the present invention, comprising a fixing plate and at least one suture, wherein the fixing plate is provided with at least two threading holes, the suture is wound to form a first coil and a second coil, and the first coil and the second coil are provided on a first side of the fixing plate; two ends of the first coil are respectively a first end and a second end, and two ends of the second coil are respectively a third end and a fourth end; the first end and the third end pass through different threading holes and are connected on the second side of the fixing plate to form a fixing portion, and the second end forms at least two fixing portion on the second side of the fixing plate by passing through the plurality of threading holes; the second end, after forming the second fixing portion, is wound around part of the first coil and part of the second coil on the first side of the fixing plate so as to form an adjusting portion. Specifically, the adjusting portion comprises at least one of a wound coil and a movable suture knot, and the fourth end is provided on a second side of the fixing plate.

In the embodiment of the present invention, the first end and the second end are respectively two ends of the first coil; the first end comprises a suture segment of one end of the first coil penetrating through the threading hole, and part of a suture segment forming the first fixing portion; the second end comprises a suture segment of the other end of the first coil penetrating through the threading hole, a suture segment forming the second fixing portion, and a suture segment after forming the second fixing portion; the suture segment after forming the second fixing portion comprises a suture segment forming the adjusting portion, a suture segment connected between the adjusting portion and the second fixing portion, and a suture segment penetrating through the threading hole after the adjusting portion and located at the second side of the fixing plate; the third end and the fourth end are respectively two ends of the second coil; the third end comprises a suture segment of one end of the second coil penetrating through the threading hole, and part of a suture segment forming the first fixing portion; the fourth end comprises a suture segment of the other end of the second coil penetrating through the threading hole, and a suture segment located at the second side of the fixing plate; the first fixing portion comprises a suture segment located at the second side of the fixing plate, and suture segments of two ends of the first fixing portion respectively penetrating through the treading holes; and the second fixing portion comprises a suture segment located at the second side of the fixing plate, and suture segments of two ends of the second fixing portion respectively penetrating through the threading holes.

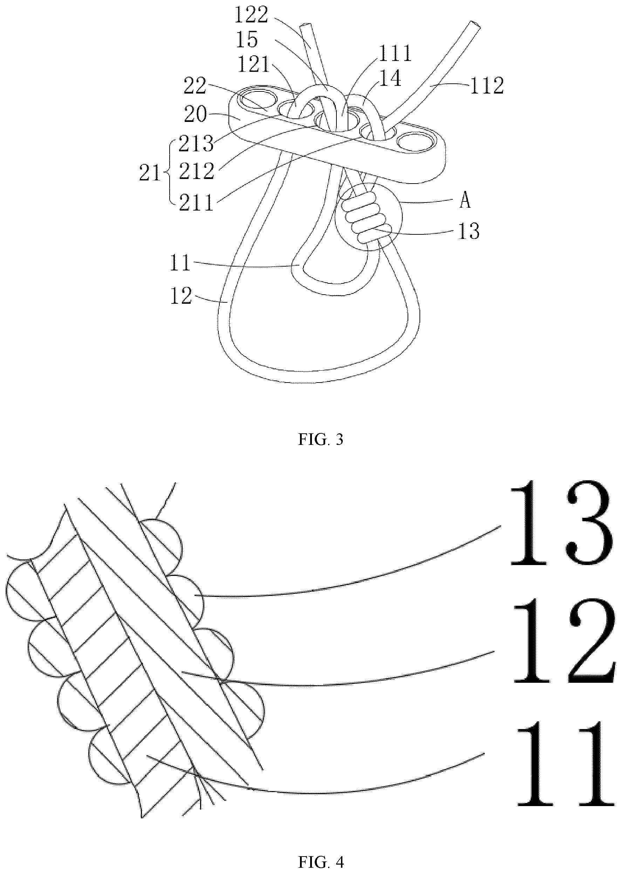

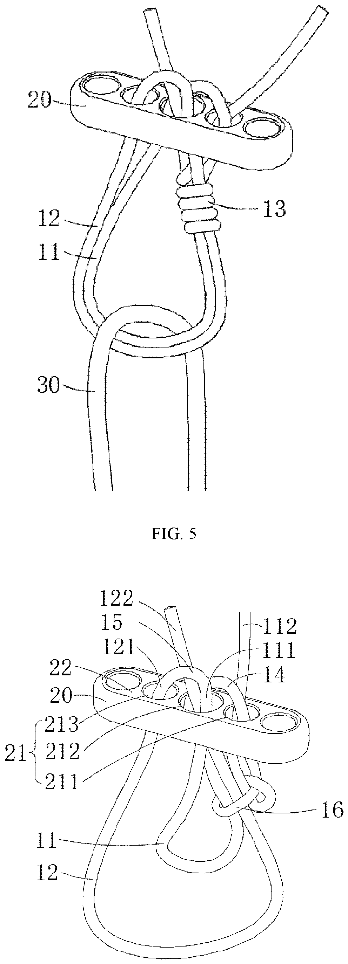

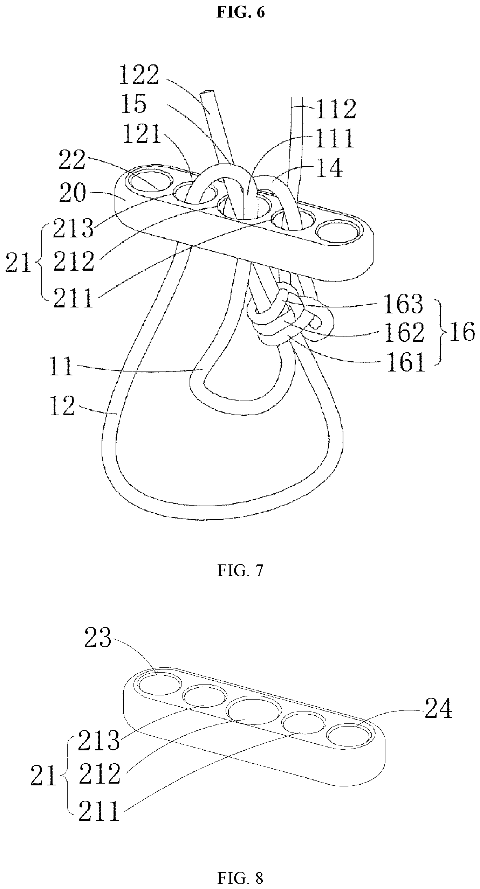

FIG. 3 is a structure diagram of a first loop of an embodiment of the present invention; FIG. 4 is a sectional diagram of A in FIG. 3 along a direction of the first and second coils being wound; FIG. 5 is a diagram of a use state of a loop in an embodiment of the present invention; FIG. 6 is a structure diagram of a second loop in an embodiment of the present invention; and FIG. 7 is a structure diagram of a third loop in an embodiment of the present invention.

Specifically, referring to FIG. 3, FIG. 4, FIG. 5, FIG. 6 and FIG. 7, the loop comprises a fixing plate 20 and at least one suture (not shown in figure); the fixing plate 20 is provided with at least two threading holes 21, the suture (not shown in figure) is wound to form a first coil 11 and a second coil 12, and the first coil 11 and the second coil 12 are provided on a first side (not shown in figure) of the fixing plate 20, so that the first coil 11 and the second coil 12 can share the pulling force of the graft 30 when fixing the graft 30, the overall stress strength of the loop is increased, and the loop is firmer, can meet clinical requirements of structural mechanics, and can be suitable for the grafts 30 with different pulling force; the two ends of the first coil 11 are respectively a first end 111 and a second end 112, and the two ends of the second coil 12 are respectively a third end 121 and a fourth end 122; the first end 111 and the third end 121 pass through different threading holes 21 and are connected on the second side 22 of the fixing plate 20 to form a first fixing portion 15, so that the first fixing portion 15 can automatically slide on the fixing plate 20 under the action of the graft 30 to adjust length of the first coil 11 and the length of the second coil 12, and the length of the first coil 11 is the same as the length of the second coil 12, thus guaranteeing that the first coil 11 and the second coil 12 can share the pulling force of the graft 30, and no redundant calculation is required during surgery, the surgery time is shortened, and the surgical risk is reduced; the second end 112 forms at least one second fixing portion 14 on the second side 22 of the fixing plate 20 by passing through the plurality of threading holes 21, and the second end 112, after forming the second fixing portion 14, is wound around part of the first coil 11 and part of the second coil 12 on the first side (not shown in figure) of the fixing plate 20 to form an adjusting portion (not shown in figure), so that the first coil 11 and the second coil 12 are connected to the fixing plate 20, and the length of the first coil 11 and the length of the second coil 12 can be adjusted by adjusting the adjusting portion (not shown in figure) to enable a fourth end 122 to slide and stretch under the action of artificial external force, i.e., the total length of the first coil 11 and the second coil 12, no redundant calculation is required during surgery, and the surgery time is shortened; when the graft 30 is fixed by the first coil 11 and the second coil 12, under the action of the pulling force of the graft 30, the second end 112 pulls the adjusting portion (not shown in figure) to contract and lock the first coil 11 and the second coil 12, so that the fourth end 122 cannot slide and stretch to adjust the length of the first coil 11 and the length of the second coil 12, and the second end 112 can automatically lock the first coil 11 and the second coil 12 without external force operation, the first coil 11 and the second coil 12 can be effectively prevented from retreating, the stability and safety of the loop in clinical use are guaranteed, the smooth surgery is ensured, and the surgical risk is reduced.

In some embodiments of the present invention, the adjusting portion comprises at least one of a wound coil and a movable suture knot, so that the length of the first coil and the length of the second coil can be adjusted by adjusting the adjusting portion to make the fourth end slide and stretch under the action of artificial external force; moreover, when the graft is fixed by the first coil and the second coil, the second end can automatically lock the first coil and the second coil without external force operation, the first coil and the second coil can be effectively prevented from retreating, the stability and safety of the loop in clinical use are guaranteed, the smooth surgery is ensured, and the surgical risk is reduced.

In some embodiments of the present invention, referring to FIG. 3, FIG. 4 and FIG. 5, the adjusting portion comprises at least one wound coil 13, the second end 112, after forming the second fixing portion 14, is wound around part of the first coil 11 and part of the second coil 12 on the first side (not shown in figure) of the fixing plate 20 so as to form the wound coil 13, the wound coil 13 is stressed more uniformly, so that the second end 112 can pull the wound coil 13 easier to contract and lock the first coil 11 and the second coil 12 when subjected to the action of the pulling force of the graft.

In some other embodiments of the present invention, the adjusting portion comprises at least one wound coil, the second end, after forming the second fixing portion, is wound around part of the first coil and part of the second coil on the first side of the fixing plate so as to form the wound coil; the adjusting portion further comprises a first movable suture knot, the second end, after forming the wound coil, is further wound around part of the first coil and part of the second coil so as to form the first movable suture knot; the first movable suture knot is provided to effectively prevent the wound coil from loosening, and the first coil and the second coil are further locked to prevent the first coil and the second coil from retreating.

In yet some other embodiments of the present invention, referring to FIG. 6 and FIG. 7, the adjusting portion comprises at least one second movable suture knot 16, the second end 112, after forming the second fixing portion 14, is wound around part of the first coil 11 and part of the second coil 12 on the first side (not shown in figure) of the fixing plate 20 to form the second movable suture knot 16; when subjected to the pulling force of the graft, the second end 112 pulls the second movable suture knot 16 to contract to make part of the first coil 11 and part of the second coil 12 deform, thus increasing the friction force between the second movable suture knot 16 and part of the first coil 11 as well as part of the second coil 12, and effectively preventing the first coil 11 and the second coil 12 from retreating under the action of the pulling force of the graft; and the second movable suture knot 16 has a better anti-retreating effect on the first coil 11 and the second coil 12.

In some specific embodiments of the present invention, referring to FIG. 6, the adjusting portion comprises one second movable suture knot 16, which is simple in structure.

In some other specific embodiments of the present invention, referring to FIG. 7, the adjusting portion comprises three second movable suture knots 16, respectively being a second movable suture knot I 161, a second movable suture knot II 162 and a second movable suture knot III 163, which have better anti-retreating effect.

In some embodiments of the present invention, the second end 112, after forming the adjusting portion, is compressed between any one of the first fixing portion 15 and the second fixing portion 14 and the fixing plate 20, so that the first fixing portion 15 or the second fixing portion 14 can automatically contract to compress and fix the second end 112 when the graft is fixed by the first coil 11 and the second coil 12, the adjusting portion is effectively prevented from loosening, and the anti-retreating effect of the first coil 11 and the second coil 12 is better.

In some embodiments of the present invention, the second end 112, after forming the adjusting portion, is compressed between the second fixing portion 14 and an end face of the second side of the fixing plate 20, i.e., the threading hole through which the second end 112 after forming the adjusting portion passes is different from the threading holes through which the two ends of the second fixing portion 14 penetrate, thus making the second end 112 be compressed between the second fixing portion 14 and the end face of the second side of the fixing plate 20 after passing through the corresponding threading hole, and further increasing the friction force between the second end 112 and the fixing plate 20 to increase the anti-retreating effect.

In some other specific embodiments of the present invention, referring to FIG. 3, FIG. 6 and FIG. 7, the second end 112, after forming the adjusting portion, is compressed between the second fixing portion 14 and the threading hole 21 of the fixing plate 20, i.e., the threading hole through which the second end 112 after forming the adjusting portion passes is the same as the threading hole through which one end of the second fixing portion 14 penetrates, thus making the second end 112 be compressed between the second fixing portion 14 and the threading hole of the fixing plate 20 when passing through the corresponding threading hole. The providing of the number of the threading hole can be reduced, and thus the size of the fixing plate 20 is reduced.

In some further specific embodiments of the present invention, the second end 112, after forming the adjusting portion, is compressed between the first fixing portion 15 and the end face of the second side of the fixing plate 20, or the second end 112, after forming the adjusting portion, is compressed between the first fixing portion 15 and the threading hole 21 of the fixing plate 20. Unnecessary details are not given herein for the specific providing mode.

In some specific embodiments of the present invention, referring to FIG. 3, the fixing plate 20 comprises three threading holes 21, respectively, a first threading hole 211, a second threading hole 212, and a third threading hole 213; two ends of the second fixing portion 14 respectively penetrate through the first threading hole 211 and the second threading hole 212, the second end 112, after forming the wound coil 13, passes through the first threading hole 211, thus making the second end 112, after forming the wound coil 13, be compressed between the second fixing portion 14 and the first threading hole 211 of the fixing plate 20.

In some other specific embodiments of the present invention, referring to FIG. 6 and FIG. 7, the fixing plate 20 comprises three threading holes 21, respectively the first threading hole 211, the second threading hole 212, and the third threading hole 213; two ends of the second fixing portion 14 penetrate through the first threading hole 211 and the second threading 212, respectively; the second end 112, after forming the second movable suture knot 16, passes through the first threading hole 211, thus making the second end 112, after forming the second movable suture knot 16, be compressed between the second fixing portion 14 and the first threading hole 211 of the fixing plate 20.

In some embodiments of the present invention, the adjusting portion is arranged close to the fourth end, and the adjusting portion is arranged to be in contact with the end face of the first side of the fixing plate; on the one hand, the fourth end can be better fixed to prevent the fourth end from falling to the first side of the fixing plate; on the other hand, when the length of the first coil and the length of the second coil are adjusted by adjusting the adjusting portion under the action of artificial external force to make the fourth end slide and stretch, the adjusting portion is in contact with the end face of the first side of the fixing plate, i.e., the adjusting portion directly abuts against the end face of the first side of the fixing plate, the operation of pulling the adjusting portion to move to abut against the end face of the first side of the fixing plate is avoided, so that the medical staff can rapidly and accurately judge the length of the first coil and the length of the second coil, and the medical staff is prevented from misjudging.

In some embodiments of the present invention, referring to FIG. 3, FIG. 5, FIG. 6 and FIG. 7, wherein the first coil 11 and the second coil 12 are woven by one continuous suture of at least one suture (not shown in figure) passing through the threading holes 21 of the fixing plate 20, i.e., the first fixing portion 15 is continuous, so that the suture segment between the first coil 11 and the second coil 12 is continuous, firmer and more durable, thus preventing the joint of the first coil 11 and the second coil 12, i.e., the first fixing portion 15, from being broken to affect the use.

In some other embodiments of the present invention, the first coil and the second coil are respectively woven by two sutures of the at least one suture passing through the threading holes of the fixing plate; the first end and the third end pass through different threading holes and are fixedly connected on the second side of the fixing plate, i.e., the first end and the third end are fixedly connected after passing through different threading holes to form the first fixing portion, and the weaving of the loop is simple and convenient.

In some embodiments of the present invention, the first end is fixedly connected to the third end through bonding mode or a binding mode to form the first fixing portion.

In some other embodiments of the present invention, the first end and the third end are arranged to be of a hard knot structure to achieve fixed connection, thus forming the first fixing portion.

In some further embodiments of the present invention, the first end is woven into the suture nested in the third end to achieve fixed connection so as to form the first fixing portion, or the third end is woven into the suture nested in the first end to achieve fixed connection so as to form the first fixing portion.

In some embodiments of the present invention, the fourth end is compressed between any one of the first fixing portion and the second fixing portion and the fixing plate, when the graft is fixed by the first coil and the second coil, the first fixing portion or the second fixing portion can automatically contract to compress and fix the fourth end, thus effectively preventing the fourth end from falling to the first side of the fixing plate and preventing the fourth end from sliding under the acting force of the graft; the first coil and the second coil can be further prevented from retreating, and the anti-retreating effect is better.

In some specific embodiments of the present invention, the fourth end 122 is compressed between the first fixing portion 15 and the end face of the second side of the fixing plate 20, i.e., the threading hole through which the fourth end 122 passes is different from the threading holes through which two ends of the first fixing portion 15 penetrate, thus making the fourth end 122, after passing through the corresponding threading hole, be compressed between the first fixing portion 15 and the end face of the second side of the fixing plate 20, the friction force between the fourth end 122 and the fixing plate 20 is further is further increased, and the anti-retreating effect is better.

In some other specific embodiments of the present invention, the fourth end 122 is compressed between the first fixing portion 15 and the threading hole 21 of the fixing plate 20, i.e., the threading hole through which the fourth end 122 passes is the same as the threading hole through which one end of the first fixing portion 15 penetrates, thus making the fourth end 122 be compressed between the first fixing portion 15 and the threading hole of the fixing plate 20 when passing through the corresponding threading hole; the providing of the number of the threading hole can be reduced, and thus the size of the fixing plate 20 is reduced.

In some further specific embodiments of the present invention, the fourth end 122 is compressed between the second fixing portion 14 and the end face of the second side 22 of the fixing plate 20, or the fourth end 122 is compressed between the second fixing portion 14 and the threading hole 21 of the fixing plate 20, unnecessary details are not given herein.

In some embodiments of the present invention, the suture is any one of a silk suture, a catgut suture, a chemical synthesis suture, and a pure natural collagen suture, and the suture conforming to stress strength is selected according to the clinic need for stress.

In some embodiments of the present invention, the suture is any one of a polyethylene surgical suture, a nylon suture, a polyester suture, a polyamide suture, and a polypropylene suture.

FIG. 8 is a structure diagram of a fixing plate of the embodiment of the present invention. In some better embodiments of the present invention, referring to FIG. 8, the fixing plate (not shown in figure) is further provided with at least one traction hole 23, and the traction hole 23 is provided to facilitate the providing of a traction rope to pull the loop to a correct position in clinic. In some preferred embodiments of the present invention, the threading hole can be temporarily taken as a traction hole for use.

In some better embodiments of the present invention, referring to FIG. 8, the fixing plate (not shown in figure) is further provided with at least one loop overturning hole 24, and the loop overturning hole 24 is provided to facilitate the providing of a loop overturning line to overturn and adjust the fixing plate to a correct angle and position in clinic. In some preferred embodiments of the present invention, the threading hole can be temporarily taken as a loop overturning line for use.

In some embodiments of the present invention, different threading holes of the fixing plate have the same aperture.

In some embodiments of the present invention, part or all of the threading holes of the fixing plate have different apertures to facilitate a plurality of suture segments to pass through the threading hole with large aperture.

In some embodiments of the present invention, the fixing plate is any one of a titanium plate and poly-ether-ether-ketone (PEEK).

In some embodiments of the present invention, the fixing plate comprises two threading holes, two ends of the first fixing portion respectively penetrate through the two threading holes, two ends of the second fixing portion respectively penetrate through the two threading holes; and when the fixing plate comprises two threading holes, the fixing plate is provided with the least threading hole, and thus the size of the fixing plate is reduced. Further, in some other embodiments of the present invention, the two ends of the first fixing portion are provided across at least one of the threading holes, and the two ends of the second fixing portion are provided across at least one of the threading holes, i.e., at least one threading hole is further provided between two threading holes through which the two ends of the first fixing portion penetrate, and at least one threading hole is further provided between two threading holes through which the two ends of the second fixing portion penetrate, thus increasing the friction force between the first fixing portion and the fixing plate and between the second fixing portion and the fixing plate, and increasing the stress strength of the first coil and the second coil for fixing the graft.

In some other embodiments of the present invention, the fixing plate comprises three threading holes, the two ends of the first fixing portion respectively penetrate through different threading holes, and the two ends of the second fixing portion respectively penetrate through different threading holes; referring to FIG. 3, FIG. 6 and FIG. 7, the fixing plate 20 comprises three threading holes 21, respectively the first threading hole 211, the second threading hole 212 and the third threading hole 213 which are arranged in sequence; the two ends of the second fixing portion 14 respectively penetrate through the first threading hole 211 and the second threading hole 212, the two ends of the first fixing portion 15 respectively penetrate through the second threading hole 212 and the third threading hole 213, wherein the aperture of the second threading hole 212 is greater than the aperture of the first threading hole 211 and the aperture of the third threading hole 213 for the passing of the plurality of suture segments, and the aperture of the first threading hole 211 is equal to the aperture of the third threading hole 213. Further, in some other embodiments of the present invention, at least one threading hole is further provided between the first threading hole 211 and the second threading hole 212, i.e., the two ends of the second fixing portion are provided across at least one of the threading holes; and at least one threading hole is further provided between the second threading hole 212 and the third threading hole 213, i.e., the two ends of the first fixing portion are provided across at least one of the threading holes.

In some further embodiments of the present invention, the fixing plate comprises four threading holes, two ends of the first fixing portion respectively penetrate through two threading holes of the four threading holes, and two ends of the second fixing portion respectively penetrate through another two threading holes of the four threading holes. Further, in some other embodiments of the present invention, two ends of the first fixing portion respectively penetrate through two threading holes of the four threading holes, and the second end sequentially penetrate through the four threading holes to form two second fixing portions on the second side of the fixing plate. Furthermore, in some other embodiments of the present invention, at least one threading hole is provided between any two threading holes of the four threading holes.

In some embodiments of the present invention, a manufacturing method of a loop is provided, comprising:
S0, providing a fixing plate and at least one suture, wherein the fixing plate is provided with at least two threading holes; and
S1, winding the suture to form a first coil and a second coil, and providing the first coil and the second coil on a first side of the fixing plate, and threading a first end of the first coil and a third end of the second coil through different threading holes and connecting the first end of the first coil to the third end of the second coil on a second side of the fixing plate to form a first fixing portion, and after threading the second end of the first coil through the plurality of threading holes to form at least one second fixing portion on the second side of the fixing plate, winding the second end around part of the first coil and part of the second coil at the first side of the fixing plate to form an adjusting portion, thus forming the loop.

In some embodiments of the present invention, after the step S1, the method further comprises: compressing the second end after forming the adjusting portion between any one of the first fixing portion and the second fixing portion and the fixing plate.

In some embodiments of the present invention, after the step S1, the method further comprises: compressing the fourth end between any one of the first fixing portion and the second fixing portion and the fixing plate.

In some embodiments of the present invention, when the first coil and the second coil are woven by one continuous suture of at least one suture passing through the threading holes of the fixing plate, the step S1 specifically comprises:
S101, winding the suture to form the first coil and the second coil, and arranging the first coil and the second coil on the first side of the fixing plate, and forming a first fixing portion on the second side of the fixing plate; and
S102, after threading the second end of the first coil through the plurality of threading holes to form at least one second fixing portion on the second side of the fixing plate, winding the second end around part of the first coil and part of the second coil at the first side of the fixing plate to form an adjusting portion.

Wherein the first fixing portion can be formed on the second side of the fixing plate in the process of winding the suture to form the first coil and the second coil in the step S101.

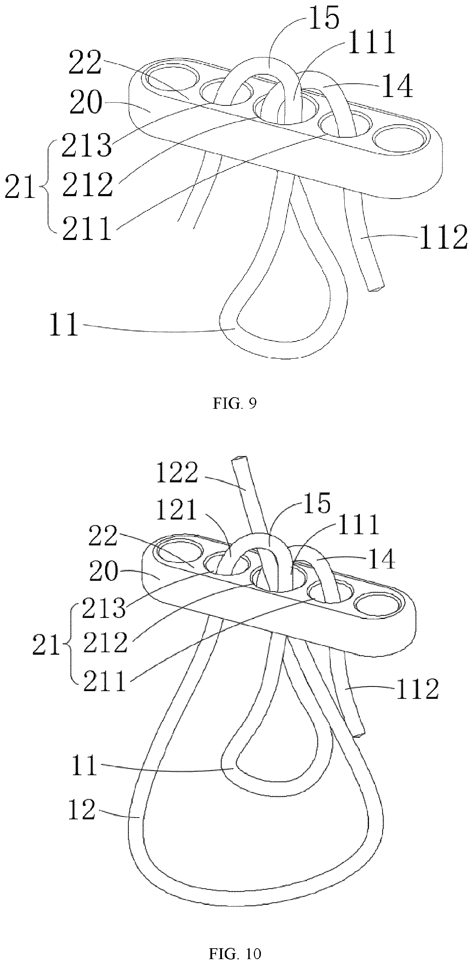

FIG. 9 is a structure diagram formed in a manufacturing process of a loop of an embodiment of the present invention; and FIG. 10 is another structure diagram formed in the manufacturing process of the loop of the embodiment of the present invention.

In some specific embodiments of the present invention, referring to FIG. 3, FIG. 9 and FIG. 10, a manufacturing method of a loop is provided, comprising:
S100, providing a fixing plate 20 and a continuous suture (not shown in figure), wherein the fixing plate 20 is provided with at least three threading holes 21, respectively a first threading hole 211, a second threading hole 212, and a third threading hole 213 which are arranged in sequence;
S101, after partially threading the continuous suture (not in figure) through the second threading hole 212 to form a first coil 11 on a first side of the fixing plate 20, threading the first end 111 of the first coil 11 through the third threading hole 213 after winding the first end of the first coil to form a first fixing portion 15 on a second side 22 of the fixing plate 20, and then winding the first end of the first coil to form a second coil 12 on the first side (not shown in figure) of the fixing plate 20, and then threading the fourth end 122 of the second coil 12 from the first side (not shown in figure) of the fixing plate 20 to the second side 22 of the fixing plate 20 through the second threading hole 212;
S102, threading the second end 112 of the first coil 11 through the first threading hole 211 after winding the second end of the first coil from the second side 22 of the fixing plate 20 to form a second fixing portion 14, and then winding the second end 112 around part of the first coil 11 and part of the second coil 12 on the first side (not shown in figure) of the fixing plate 20 so as to form at least one wound coil 13; and
S103, threading the second end 112 through the first threading hole 211 from the first side (not shown in figure) of the fixing plate 20, and compressing the second end between the second fixing portion 14 and the first threading hole 211.

In some other specific embodiments of the present invention, the step S101 comprises: after threading one end of the continuous suture (not shown in figure) from the second side 22 of the fixing plate 20 to the first side (not shown in figure) of the fixing plate 20 through the second threading hole 212 to be wound to form a second coil 12, threading the third end 121 of the second coil 12 through the third threading hole 213 to be wound to form a first fixing portion 15 on the second side 22 of the fixing plate 20, and then threading the third end of the second coil to the first side (not shown in figure) of the fixing plate 20 through the second threading hole 212 to be wound to form the first coil 11. At the moment, in the step S102, the second end 112 of the first coil 11 needs to be wound to form a second fixing portion 14 on the second side 22 of the fixing plate 20 by passing through the second threading hole 212 from the first side (not shown in figure) of the fixing plate 20 and then to pass through the first threading hole 211, and the second end 112 is wound around part of the first coil 11 and part of the second coil 12 on the first side (not show in figure) of the fixing plate 20 so as to form at least one wound coil 13.

In some further specific embodiments of the present invention, referring to FIG. 6, the step S102 comprises: after threading the second end 112 of the first coil 11 through the first threading hole 211 from the second side 22 of the fixing plate 20, winding the second end 112 around part of the first coil 11 and part of the second coil 12 on the first side (not shown in figure) of the fixing plate 20 to form at least one second movable suture knot 16.

In still some specific embodiments of the present invention, referring to FIG. 7, the step S102 comprises: after threading the second end 112 of the first coil 11 through the first threading hole 211 from the second side 22 of the fixing plate 20, winding the second end 112 around part of the first coil 11 and part of the second coil 12 on the first side (not shown in figure) of the fixing plate 20 to sequentially form a second movable suture I 161, a second movable suture knot II 162, and a second movable suture knot III 163.

In some embodiments of the present invention, when the first coil and the second coil are respectively woven by two sutures of the at least one suture passing through the threading holes of the fixing plate, the step S1 specifically comprises:
S111: winding one of the sutures to form the first coil and winding the other of the sutures to form the second coil, and providing the first coil and the second coil on the first side of the fixing plate;
S112: after threading the first end of the first coil and the third end of the second coil through different threading holes, connecting the first end of the first coil to the third end of the second coil on the second side of the fixing plate to form the first fixing portion; and
S113, after threading the second end of the first coil through the plurality of threading holes to form at least one second fixing portion on the second side of the fixing plate, winding the second end around part of the first coil and part of the second coil on the first side of the fixing plate to form the adjusting portion.

In some specific embodiments of the present invention, a manufacturing method of a loop is provided, comprising:
S110, providing a fixing plate 20 and two sutures (not shown in figure), wherein the fixing plate 20 is provided with at least three threading holes 21, respectively being a first threading hole 211, a second threading hole 212, and a third threading 213 which are arranged in sequence;
S111, threading part of one of the sutures (not shown in figure) through the second threading hole 212 to form the first coil 11 on the first side of the fixing plate 20, and threading one end of another of the sutures (not shown in figure) from a second side 22 of the fixing plate 20 to a first side (not shown in figure) of the fixing plate 20 through the first threading hole 212 to be wound to form the second coil 12;
S112, after threading a third end of the second coil 12 through the third threading hole 213, connecting a first end 111 of the first coil 11 to a third end 121 of the second coil 12 on the second side 22 of the fixing plate 20 to form a first fixing portion 15; and
S113, threading the second end 112 of the first coil 11 through the first threading hole 211 after winding the second end of the first coil to form a second fixing portion 14 on the second side 22 of the fixing plate 20, and then winding the second end 112 around part of the first coil 11 and part of the second coil 12 on the first side (not shown in figure) of the fixing plate 20 so as to form a wound coil 13.

While the embodiments of the present invention have been described in detail, it will be apparent to those skilled in the art that various variations can be made to the embodiments within the scope of the appended claims. Furthermore, the present invention described herein is susceptible to other embodiments and may be embodied or carried out in various ways within the scope of the appended claims.

## Claims

1. A loop, comprising at least one suture, **characterized in that**, the loop further comprises a fixing plate (20), wherein the fixing plate (20) is provided with at least two threading holes (21), the suture is wounded to form a first coil (11) and a second coil (12), and the first coil (11) and the second coil (12) are provided on a first side of the fixing plate (20); two ends of the first coil (11) are respectively a first end (111) and a second end (112) , and two ends of the second coil (12) are respectively a third end (121) and a fourth end (122); the first end (111) and the third end (121) pass through the different threading holes (21) and are connected on a second side (22) of the fixing plate (20) to form a first fixing portion (15), the second end (112) forms at least one second fixing portion (14) on the second side (22) of the fixing plate (20) by passing through the plurality of threading holes (21), and the second end (112), after forming the second fixing portion (14), is wound around part of the first coil (11) and part of the second coil (12) at the first side of the fixing plate (20) to form an adjusting portion.

2. The loop according to claim 1, **characterized in that**, the adjusting portion comprises at least one of a wound coil (13) and a movable suture knot.

3. The loop according to claim 1, **characterized in that**, the adjusting portion comprises at least one wound coil (13), the second end (112), after forming the second fixing portion (14), is wound around part of the first coil (11) and part of the second coil (12) at the first side of the fixing plate (20) to form the wound coil (13).

4. The loop according to claim 3, **characterized in that**, the adjusting portion further comprises a first movable suture knot, the second end (112), after forming the wound coil (13), is further wounded around part of the first coil (11) and part of the second coil (12) to form the first movable suture knot.

5. The loop according to claim 4, **characterized in that**, the adjusting portion comprises at least one second movable suture knot (16), and the second end (112), after forming the second fixing portion (14), is wounded around part of the first coil (11) and part of the second coil (12) at the first side of the fixing plate (20) to form the second movable suture knot (16).

6. The loop according to any one of claims 1-5, **characterized in that**, the second end (112), after forming the adjusting portion, is compressed between any one of the first fixing portion (15) and the second fixing portion (14) and the fixing plate (20).

7. The loop according to claim 1, **characterized in that**, the adjusting portion is arranged close to the fourth end (122), and the adjusting portion is arranged to be in contact with an end face of the first side of the fixing plate (20).

8. The loop according to claim 1, **characterized in that**, the first coil (11) and the second coil (12) are woven by one continuous suture of at least one suture through the threading holes (21) of the fixing plate (20).

9. The loop according to claim 1, **characterized in that**, the first coil (11) and the second coil (12) are respectively woven by two sutures of at least one of sutures through the threading holes (21) of the fixing plate (20).

10. The loop according to claim 1, **characterized in that**, the fourth end (122) is compressed between any one of the first fixing portion (15) and the second fixing portion (14) and the fixing plate (20).

11. A manufacturing method for a loop, **characterized in that** it comprises:
S0, providing a fixing plate (20) and at least one suture, wherein the fixing plate (20) is provided with at least two threading holes (21); and
S1, winding the suture to form a first coil (11) and a second coil (12), and providing the first coil (11) and the second coil (12) on a first side of the fixing plate (20), and threading a first end (111) of the first coil (11) and a third end (121) of the second coil (12) through different threading holes (21) and connecting the first end (111) of the first coil (11) and the third end (121) of the second coil (12) on a second side of the fixing plate (20) to form a first fixing portion (15), and threading a second end (112) of the first coil (11) through the plurality of threading holes (21) to form at least one second fixing portion (14) on the second side of the fixing plate (20), and then winding the second end (112) around part of the first coil (11) and part of the second coil (12) at the first side of the fixing plate (20) to form an adjusting portion, thus forming the loop according to any one of claims 1-10.

12. The manufacturing method for the loop according to claim 11, **characterized in that**, when the first coil (11) and the second coil (12) are woven by one continuous suture of at least one suture through the threading holes (21) of the fixing plate (20), the step S1 specifically comprises:
S101, winding the continuous suture to form the first coil (11) and the second coil (12), and providing the first coil (11) and the second coil (12) on the first side of the fixing plate (20), and forming a first fixing portion (15) on the second side of the fixing plate (20); and
S102, after threading the second end (112) of the first coil (11) through the plurality of threading holes (21) to form at least one second fixing portion (14) on the second side of the fixing plate (20), winding the second end (112) around part of the first coil (11) and part of the second coil (12) at the first side of the fixing plate (20) to form an adjusting portion.

13. The manufacturing method for the loop according to claim 11, **characterized in that**, when the first coil (11) and the second coil (12) are respectively woven by two sutures of at least one suture through the threading holes (21) of the fixing plate (20), the step S1 specifically comprises:
S111: winding one of the sutures to form the first coil (11) and winding the other of the sutures to form the second coil (12), and providing the first coil (11) and the second coil (12) on the first side of the fixing plate (20);
S112: after threading the first end (11 1)of the first coil (11) and the third end (121) of the second coil (12) through different threading holes (21), connecting the first end (111) of the first coil (11) to the third end (121) of the second coil (12)on the second side of the fixing plate (20) to form the first fixing portion (15); and
S113, after threading the second end (112) of the first coil (11) through the plurality of threading holes (21) to form at least one second fixing portion (14) on the second side of the fixing plate (20), winding the second end (112) around part of the first coil (11) and part of the second coil (12) on the first side of the fixing plate (20) to form the adjusting portion.

## Patentansprüche

1. Schleife, die mindestens eine Naht umfasst, **dadurch gekennzeichnet, dass** die Schleife ferner eine Befestigungsplatte (20) umfasst, wobei die Befestigungsplatte (20) mit mindestens zwei Einfädellöchern (21) versehen ist, die Naht so gewickelt ist, um eine erste Spule (11) und eine zweite Spule (12) zu bilden, und die erste Spule (11) und die zweite Spule (12) auf einer ersten Seite der Befestigungsplatte (20) vorgesehen sind; wobei die beiden Enden der ersten Spule (11) jeweils ein erstes Ende (111) und ein zweites Ende (112) sind und die beiden Enden der zweiten Spule (12) jeweils ein drittes Ende (121) und ein viertes Ende (122) sind; wobei das erste Ende (111) und das dritte Ende (121) durch die verschiedenen Einfädellöcher (21) durchgeführt und auf einer zweiten Seite (22) der Befestigungsplatte (20) verbunden sind, um einen ersten Befestigungsabschnitt (15) zu bilden, das zweite Ende (112) auf der zweiten Seite (22) der Befestigungsplatte (20) mindestens einen zweiten Befestigungsabschnitt (14) bildet, indem es durch die Vielzahl von Einfädellöchern (21) durchgeführt wird, und wobei nach Bildung des zweiten Befestigungsabschnitts (14) das zweite Ende (112) um einen Teil der ersten Spule (11) und einen Teil der zweiten Spule (12) an der ersten Seite der Befestigungsplatte (20) gewickelt wird, um einen Anpassungsabschnitt zu bilden.

2. Schleife nach Anspruch 1, **dadurch gekennzeichnet, dass** der Anpassungsabschnitt mindestens eine gewickelte Spule (13) und einen bewegbaren Nahtknoten umfasst.

3. Schleife nach Anspruch 1, **dadurch gekennzeichnet, dass** der Anpassungsabschnitt mindestens eine gewickelte Spule (13) umfasst, wobei nach Bildung des zweiten Befestigungsabschnitts (14) das zweite Ende (112) um einen Teil der ersten Spule (11) und einen Teil der zweiten Spule (12) an der ersten Seite der Befestigungsplatte (20) gewickelt wird, um die gewickelte Spule (13) zu bilden.

4. Schleife nach Anspruch 3, **dadurch gekennzeichnet, dass** der Anpassungsabschnitt ferner einen ersten bewegbaren Nahtknoten umfasst, wobei nach Bildung der gewickelten Spule (13) das zweite Ende (112) ferner um einen Teil der ersten Spule (11) und einen Teil der zweiten Spule (12) gewickelt wird, um den ersten bewegbaren Nahtknoten zu bilden.

5. Schleife nach Anspruch 4, **dadurch gekennzeichnet, dass** der Anpassungsabschnitt mindestens einen zweiten bewegbaren Nahtknoten (16) umfasst, und wobei nach Bildung des zweiten Befestigungsabschnitts (14) das zweite Ende (112) um einen Teil der ersten Spule (11) und einen Teil der zweiten Spule (12) an der ersten Seite der Befestigungsplatte (20) gewickelt wird, um den zweiten bewegbaren Nahtknoten (16) zu bilden.

6. Schleife nach einem der Ansprüche 1 bis 5, **dadurch gekennzeichnet, dass** nach Bildung des Anpassungsabschnitts das zweite Ende (112) zwischen einem beliebigen von dem ersten Befestigungsabschnitt (15) beziehungsweise dem zweiten Befestigungsabschnitt (14) und der Befestigungsplatte (20) gepresst wird.

7. Schleife nach Anspruch 1, **dadurch gekennzeichnet, dass** der Anpassungsabschnitt in unmittelbarer Nähe des vierten Endes (122) angeordnet ist, und wobei der Anpassungsabschnitt so angeordnet ist, dass er in Kontakt mit einer Endfläche der ersten Seite der Befestigungsplatte (20) ist.

8. Schleife nach Anspruch 1, **dadurch gekennzeichnet, dass** die erste Spule (11) und die zweite Spule (12) mittels einer kontinuierlichen Naht aus mindestens einer Naht durch die Einfädellöcher (21) der Befestigungsplatte (20) gewoben sind.

9. Schleife nach Anspruch 1, **dadurch gekennzeichnet, dass** die erste Spule (11) und die zweite Spule (12) jeweils mittels zweier Nähte aus mindestens einer der Nähte durch die Einfädellöcher (21) der Befestigungsplatte (20) gewoben sind.

10. Schleife nach Anspruch 1, **dadurch gekennzeichnet, dass** das vierte Ende (122) zwischen einem beliebigen von dem ersten Befestigungsabschnitt (15) beziehungsweise dem zweiten Befestigungsabschnitt (14) und der Befestigungsplatte (20) gepresst wird.

11. Herstellungsverfahren für eine Schleife, **dadurch gekennzeichnet, dass** es die folgenden Schritte umfasst:
S0, Bereitstellen einer Befestigungsplatte (20) und mindestens einer Naht, wobei die Befestigungsplatte (20) mit mindestens zwei Einfädellöchern (21) versehen ist; und
S1, Aufwickeln der Naht, um eine erste Spule (11) und eine zweite Spule (12) zu bilden, und Bereitstellen der ersten Spule (11) und der zweiten Spule (12) auf einer ersten Seite der Befestigungsplatte (20), und Einfädeln eines ersten Endes (111) der ersten Spule (11) und eines dritten Endes (121) der zweiten Spule (12) durch verschiedene Einfädellöcher (21) und Verbinden des ersten Endes (111) der ersten Spule (11) und des dritten Endes (121) der zweiten Spule (12) auf einer zweiten Seite der Befestigungsplatte (20) um einen ersten Befestigungsabschnitt (15) zu bilden, und Einfädeln eines zweiten Endes (112) der ersten Spule (11) durch die Vielzahl von Einfädellöchern (21), um mindestens einen zweiten Befestigungsabschnitt (14) auf der zweiten Seite der Befestigungsplatte (20) zu bilden, und anschließend Aufwickeln des zweiten Endes (112) um einen Teil der ersten Spule (11) und einen Teil der zweiten Spule (12) auf der ersten Seite der Befestigungsplatte (20), um einen Anpassungsabschnitt zu bilden, wodurch die Schleife nach einem der Ansprüche 1 bis 10 gebildet wird.

12. Herstellungsverfahren für die Schleife nach Anspruch 11, **dadurch gekennzeichnet, dass** wenn die erste Spule (11) und die zweite Spule (12) mittels einer kontinuierlichen Naht aus mindestens einer Naht durch die Einfädellöcher (21) der Befestigungsplatte (20) gewoben sind, umfasst der Schritt S1 insbesondere:
S101, Aufwickeln des kontinuierlichen Nahts, um die erste Spule (11) und die zweite Spule (12) zu bilden, und Bereitstellen der ersten Spule (11) und der zweiten Spule (12) auf der ersten Seite der Befestigungsplatte (20), und Bilden eines ersten Befestigungsabschnitts (15) auf der zweiten Seite der Befestigungsplatte (20); und
S102, nach dem Einfädeln des zweiten Endes (112) der ersten Spule (11) durch die Vielzahl von Einfädellöchern (21), um mindestens einen zweiten Befestigungsabschnitt (14) auf der zweiten Seite der Befestigungsplatte (20) zu bilden, dann Aufwickeln des zweiten Endes (112) um einen Teil der ersten Spule (11) und einen Teil der zweiten Spule (12) auf der ersten Seite der Befestigungsplatte (20), um einen Anpassungsabschnitt zu bilden.

13. Herstellungsverfahren für die Schleife nach Anspruch 11, **dadurch gekennzeichnet, dass,** wenn die erste Spule (11) und die zweite Spule (12) jeweils mittels zweier Nähte aus mindestens einer Naht durch die Einfädellöcher (21) der Befestigungsplatte (20) gewoben sind, der Schritt S1 insbesondere umfasst:
S111: Aufwickeln eines der Nähte, um die erste Spule (11) zu bilden, und Aufwickeln des anderen der Nähte, um die zweite Spule (12) zu bilden, und Bereitstellen der ersten Spule (11) und der zweiten Spule (12) auf der ersten Seite der Befestigungsplatte (20);
S112: nach dem Einfädeln des ersten Endes (111) der ersten Spule (11) und des dritten Endes (121) der zweiten Spule (12) durch verschiedene Einfädellöcher (21), dann Verbinden des ersten Endes (111) der ersten Spule (11) mit dem dritten Ende (121) der zweiten Spule (12) auf der zweiten Seite der Befestigungsplatte (20), um den ersten Befestigungsabschnitt (15) zu bilden; und
S113: nach dem Einfädeln des zweiten Endes (112) der ersten Spule (11) durch die Vielzahl von Einfädellöchern (21), um mindestens einen zweiten Befestigungsabschnitt (14) auf der zweiten Seite der Befestigungsplatte (20) zu bilden, dann Aufwickeln des zweiten Endes (112) um einen Teil der ersten Spule (11) und einen Teil der zweiten Spule (12) auf der ersten Seite der Befestigungsplatte (20), um einen Anpassungsabschnitt zu bilden.

## Revendications

1. Boucle, comprenant au moins une suture, **caractérisée en ce que** la boucle comprend en outre une plaque de fixation (20), dans laquelle la plaque de fixation (20) est munie d'au moins deux trous de filetage (21), la suture est enroulée pour former une première bobine (11) et une deuxième bobine (12), et la première bobine (11) et la deuxième bobine (12) sont prévues sur un premier côté de la plaque de fixation (20) ; les deux extrémités de la première bobine (11) sont respectivement une première extrémité (111) et une deuxième extrémité (112), et les deux extrémités de la deuxième bobine (12) sont respectivement une troisième extrémité (121) et une quatrième extrémité (122) ; la première extrémité (111) et la troisième extrémité (121) passent à travers les différents trous de filetage (21) et sont reliées sur un deuxième côté (22) de la plaque de fixation (20) pour former une première portion de fixation (15), la deuxième extrémité (112) forme au moins une deuxième portion de fixation (14) sur le deuxième côté (22) de la plaque de fixation (20) en passant à travers la pluralité de trous de filetage (21), et la deuxième extrémité (112), une fois la deuxième portion de fixation (14) formée, est enroulée autour d'une partie de la première bobine (11) et d'une partie de la deuxième bobine (12) du premier côté de la plaque de fixation (20) pour former une portion de réglage.

2. Boucle selon la revendication 1, **caractérisée en ce que** la portion de réglage comprend au moins une d'une bobine enroulée (13) et d'un noeud de suture mobile.

3. Boucle selon la revendication 1, **caractérisée en ce que** la portion de réglage comprend au moins une bobine enroulée (13), et que la deuxième extrémité (112), une fois la deuxième portion de fixation (14) formée, est enroulée autour d'une partie de la première bobine (11) et d'une partie de la deuxième bobine (12) sur le premier côté de la plaque de fixation (20) pour former la bobine enroulée (13).

4. Boucle selon la revendication 3, **caractérisée en ce que** la portion de réglage comprend en outre un premier noeud de suture mobile, et que la deuxième extrémité (112), une fois la bobine enroulée (13) formée, est à son tour enroulée autour d'une partie de la première bobine (11) et d'une partie de la deuxième bobine (12) pour former le premier noeud de suture mobile.

5. Boucle selon la revendication 4, **caractérisée en ce que** la portion de réglage comprend au moins un deuxième noeud de suture mobile (16), et que la deuxième extrémité (112), une fois la deuxième portion de fixation (14) formée, est enroulée autour d'une partie de la première bobine (11) et d'une partie de la deuxième bobine (12) du premier côté de la plaque de fixation (20) pour former le deuxième noeud de suture mobile (16).

6. Boucle selon l'une quelconque des revendications 1 à 5, **caractérisée en ce que** la deuxième extrémité (112), une fois la portion de réglage formée, est comprimée entre l'une quelconque de la première portion de fixation (15) et de la deuxième portion de fixation (14) et la plaque de fixation (20).

7. Boucle selon la revendication 1, **caractérisée en ce que** la portion de réglage est disposée proche de la quatrième extrémité (122) et que la portion de réglage est disposée de manière à être en contact avec une face d'extrémité du premier côté de la plaque de fixation (20).

8. Boucle selon la revendication 1, **caractérisée en ce que** la première bobine (11) et la deuxième bobine (12) sont tissées par une suture continue d'au moins une suture à travers les trous de filetage (21) de la plaque de fixation (20).

9. Boucle selon la revendication 1, **caractérisée en ce que** la première bobine (11) et la deuxième bobine (12) sont respectivement tissées par deux sutures d'au moins une des sutures à travers les trous de filetage (21) de la plaque de fixation (20).

10. Boucle selon la revendication 1, **caractérisée en ce que** la quatrième extrémité (122) est comprimée entre l'une quelconque de la première portion de fixation (15) et de la deuxième portion de fixation (14) et la plaque de fixation (20).

11. Procédé de fabrication d'une boucle, **caractérisé en ce qu'**il comprend :
S0, fournir une plaque de fixation (20) et au moins une suture, dans lequel la plaque de fixation (20) est munie d'au moins deux trous de filetage (21) ; et
S1, enrouler la suture pour former une première bobine (11) et une deuxième bobine (12), et fournir la première bobine (11) et la deuxième bobine (12) sur un premier côté de la plaque de fixation (20), et enfiler une première extrémité (111) de la première bobine (11) et une troisième extrémité (121) de la deuxième bobine (12) à travers différents trous de filetage (21) et relier la première extrémité (111) de la première bobine (11) et la troisième extrémité (121) de la deuxième bobine (12) à un deuxième côté de la plaque de fixation (20) pour former une première portion de fixation (15), et enfiler une deuxième extrémité (112) de la première bobine (11) à travers la pluralité de trous de filetage (21) pour former au moins une deuxième portion de fixation (14) sur le deuxième côté de la plaque de fixation (20), puis enrouler la deuxième extrémité (112) autour d'une partie de la première bobine (11) et d'une partie de la deuxième bobine (12) sur le premier côté de la plaque de fixation (20) pour former une portion de réglage, formant ainsi la boucle selon l'une quelconque des revendications 1 à 10.

12. Procédé de fabrication de la boucle selon la revendication 11, **caractérisé en ce que** la première bobine (11) et la deuxième bobine (12) sont tissées par une suture continue d'au moins une suture à travers les trous de filetage (21) de la plaque de fixation (20), et que l'étape S1 comprend en particulier :
S 101, enrouler la suture continue pour former la première bobine (11) et la deuxième bobine (12), et fournir la première bobine (11) et la deuxième bobine (12) sur le premier côté de la plaque de fixation (20),
et former une première portion de fixation (15) sur le deuxième côté de la plaque de fixation (20) ; et
S102, une fois la deuxième extrémité (112) de la première bobine (11) enfilée à travers la pluralité de trous de filetage (21) pour former au moins une deuxième portion de fixation (14) sur le deuxième côté de la plaque de fixation (20), enrouler la deuxième extrémité (112) autour d'une partie de la première bobine (11) et d'une partie de la deuxième bobine (12) sur le premier côté de la plaque de fixation (20) pour former une portion de réglage.

13. Procédé de fabrication de la boucle selon la revendication 11, **caractérisé en ce que** la première bobine (11) et la deuxième bobine (12) sont respectivement tissées par deux sutures d'au moins une suture à travers les trous de filetage (21) de la plaque de fixation (20), et que l'étape S1 comprend en particulier :
S111 : enrouler l'une des sutures pour former la première bobine (11) et enrouler l'autre des sutures pour former la deuxième bobine (12), et fournir la première bobine (11) et la deuxième bobine (12) sur le premier côté de la plaque de fixation (20) ;
S112 : une fois la première extrémité (111) de la première bobine (11) et la troisième extrémité (121) de la deuxième bobine (12) enfilées à travers différents trous de filetage (21), relier la première extrémité (111) de la première bobine (11) à la troisième extrémité (121) de la deuxième bobine (12) sur le deuxième côté de la plaque de fixation (20) pour former la première portion de fixation (15) ; et
S113, une fois la deuxième extrémité (112) de la première bobine (11) enfilée à travers la pluralité de trous de filetage (21) pour former au moins une deuxième portion de fixation (14) sur le deuxième côté de la plaque de fixation (20), enrouler la deuxième extrémité (112) autour d'une partie de la première bobine (11) et d'une partie de la deuxième bobine (12) sur le premier côté de la plaque de fixation (20) pour former une portion de réglage.
